# EUROPÄISCHE PATENTSCHRIFT

(11) **EP 1 392 630 B1**
(45) Veröffentlichungstag und Bekanntmachung des Hinweises auf die Patenterteilung: **29.09.2004**
(21) Anmeldenummer: 02803371.0
(22) Anmeldetag: 15.11.2002
(51) Int. Cl.: C07C 39/06, C07C 37/16

(54) **SELEKTIVE HERSTELLUNG VON O-ALKYLPHENOLEN**
SELECTIVE PRODUCTION OF O-ALKYLPHENOLS
PRODUCTION SELECTIVE DE O-ALKYLPHENOLS

(30) Priorität: 21.11.2001 DE 10157073
(43) Veröffentlichungstag der Anmeldung: 03.03.2004
(73) Patentinhaber: RÜTGERS Chemicals AG, 44579 Castrop-Rauxel (DE)
(72) Erfinder: TALBIERSKY, Jörg, 46282 Dorsten (DE); FUHRMANN, Edgar, 44579 Castrop-Rauxel (DE); BRÜGGEMANN, Wolfgang, 44581 Castrop-Rauxel (DE)
(74) Vertreter: COHAUSZ & FLORACK
(86) Internationale Anmeldenummer: PCT/EP2002/012789
(87) Internationale Veröffentlichungsnummer: WO 2003/043965

(56) Entgegenhaltungen:
- DE-A- 2 756 461
- US-A- 3 737 466

## Beschreibung

Die Erfindung betrifft ein mehrstufiges Verfahren zur o-Alkylierung von Phenol durch Umsetzen von Phenol mit einem Alkanol bei erhöhter Temperatur in der Gasphase in Gegenwart eines sauren Metalloxidkatalysators.

Reine o-Alkylphenole sind wichtige Verbindungen, die in großen Mengen als Ausgangsstoff für organisch chemische Synthesen eingesetzt werden. Reines o-Kresol (2-Methylphenol) findet insbesondere Verwendung zur Herstellung von Pflanzenschut zmitteln.

o-Kresol kann durch Methylierung von Phenol mit Methanol in der Gas- oder Flüssigphase erhalten werden. Wegen der geringen Reaktivität von Methanol erfolgt die Umsetzung bei erhöhten Temperaturen in Gegenwart eines Katalysators. Als Katalysatoren werden Metalloxidkatalysatoren wie Aluminiumoxid, Siliciumoxid/Aluminium-Mischoxide und Magnesiumoxid eingesetzt. Die Wahl der Reaktionstemperatur erfolgt in Abhängigkeit von dem eingesetzten Katalysator in einem Bereich von 250 bis 460°C. So zeigt Magnesiumoxid in einem Temperaturbereich von 420 bis 460°C eine hohe Selektivität für o-Kresol, wohingegen γ-Aluminiumoxide die Methylierung von Phenol bei einer Temperatur von 200 bis 400°C katalysieren.

Bekannt ist auch die Gewinnung von o-Kresol als Nebenprodukt bei der Synthese von 2,6-Dimethylphenol und die anschließende Isolierung des o-Kresols durch zusätzliche Reinigungsschritte. Verfahren zur Erzeugung von o-Kresol mit weiteren Nachweisen sind beschrieben in H.G. Franck, J.W. Stadelhofer, INDUSTRIELLE AROMATENCHEMIE, S.170-177, Springer Verlag 1987.

Die DE 27 56 461 A1 beschreibt ein gattungsgemäßes Verfahren, das bei einer Temperatur von 250 bis 330°C mit Tonerde als Katalysator durchgeführt wird. Bei einem Verhältnis von Methanol zu Phenol von 0,5 : 1 wird eine Ausbeute an o-Kresol von bis zu 26% erzielt. Im Produkt ist ein Anteil von etwa 6% 2,6-Dimethylphenol enthalten.

Bei hohen Phenolumsätzen entstehen neben o-Kresol stets hohe Anteile an 2,6-Dimethylphenol. Problematisch bei der Methylierung von Phenol ist die selektive Gewinnung von o-Kresol. Meist treten m-Kresol und p-Kresol oder höher alkylierte Produkte in erheblichen Mengen als Nebenprodukte auf.

Allen bekannten Verfahren zur industriellen Synthese von o-Alkylphenolen gemeinsam ist der hohe Anteil an Nebenprodukten.

Der Erfindung liegt daher die Aufgabe zugrunde, ein möglichst selektives, im industriellen Maßstab durchführbares Verfahren zur Erzeugung von o-Alkylphenol bereitzustellen.

Gelöst wird diese Aufgabe durch o-Alkylierung von Phenol mit einem Alkanol bei erhöhter Temperatur in der Gasphase in Gegenwart eines Metalloxidkatalysators, in dem man die Umsetzung in mindestens zwei Reaktionsstufen durchführt und das Molverhältnis Alkanol/Phenol über das gesamte Verfahren auf einem Wert von etwa ≤ 1 hält.

Das erfindungsgemäße Verfahren kann beispielsweise in zwei bis fünf Stufen durchgeführt werden. Besonders bevorzugt erfolgt das Verfahren dreistufig. Jede Umsetzungsstufe kann in einem anderen Reaktor durchgeführt werden. Es ist aber auch möglich, mehrere Reaktionsstufen in einem Reaktor durchzuführen. Bei dieser Verfahrensweise sind in dem Reaktor mehrere räumlich voneinander getrennte Anordnungen des aktiven Katalysators untergebracht. Zwischen den Katalysatoranordnungen können Zonen mit Katalysator geringerer Aktivität oder ohne Katalysator angeordnet sein.

Die dieser Erfindung zugrunde liegenden Arbeiten haben gezeigt, dass bei der Methylierung von Phenol Anisol entsteht und o-Kresol einerseits durch Alkylierung von Phenol mit Anisol und andererseits durch intramolekulare Umlagerung des Anisols zu o-Kresol gebildet wird. Diese Erkenntnis ist neu und steht im Gegensatz zu bisherigen Erkenntnissen. Der neu aufgefundene Reaktionsverlauf ist in Fig. 1 dargestellt.

Überraschend hat sich gezeigt, dass die mehrstufige Verfahrensführung unter den zuvor genannten Bedingungen zu einer deutlich erhöhten Selektivität der Reaktion für o-Kresol und einer damit verbundenen Steigerung der Ausbeute an dieser Verbindung führt. Es wird angenommen, daß durch die bei dieser Fahrweise erforderlichen geringen lokalen Konzentrationen an Methanol und damit auch an Anisol die hohe Selektivität für o-Alkylierungen erzielt wird. Vorteilhaft ist darüber hinaus, dass die stark exotherme Reaktion durch eine Verteilung auf zwei, insbesondere drei Reaktoren, sehr viel besser zu steuern ist. Die Bildung sogenannter Hot Spots wird dabei unterdrückt.

Durch die mehrstufige Reaktionsdurchführung kann das Verhältnis Alkanol zu Phenol in jedem Reaktor oder in jeder Reaktionsstufe besonders niedrig eingestellt werden. Dadurch wird der Phenolumsatz begrenzt, und es kann eine besonders hohe Selektivität für das o-Alkylphenol, beispielsweise o-Kresol, erzielt werden. Das Molverhältnis Alkanol zu Phenol über das gesamte Verfahren wird vorzugsweise auf 0,9, besonders bevorzugt auf 0,6 oder einen dazwischen liegenden Wert eingestellt. Damit beträgt das Molverhältnis Alkanol zu Phenol bei dreistufiger Fahrweise in jeder Reaktionsstufe vorzugsweise 0,3 bis 0,2.

Alternativ dargestellt, kann der Phenolumsatz auf einen Wert von etwa 35 bis 43%, beispielsweise 38 bis 42%, eingestellt werden, um die gewünschte hohe Selektivität zu erhalten.

Zur Durchführung des erfindungsgemäßen Verfahren geeignete Katalysatoren sind saure Metalloxide und deren Gemische. Solche Metalloxide sind beispielsweise Aluminiumoxid, Siliciumoxid/Aluminium-Mischoxide und Magnesiumoxid. Besonders bevorzugt ist γ-Aluminiumoxid. Die Oberfläche der Katalysatoren ist vorzugsweise etwa 250 m²/g und mehr, besonders bevorzugt 250 bis 300 m²/g. Diese Katalysatoren werden nach bekannten Verfahren hergestellt, beispielsweise durch ammoniakalische Hydrolyse von Aluminiumnitrat und anschließender Abtrennung, Trocknung und Calcinierung des erhaltenen Niederschlags (J. Amer. Chem. Soc. 82 (1960) 2471).

Der Katalysator kann in der üblichen Form, beispielsweise als Festbett, Fließbett oder Wirbelbett, angeordnet sein. Der Katalysator ist vorzugsweise in einem Festbett angeordnet.

Erfindungsgemäß einzusetzende Alkanole sind insbesondere C₁₋₄-Alkanole, also Methanol, Ethanol, n-Propanol, Isopropanol, n-Butanol und Isobutanol.

Das erfindungsgemäße Verfahren kann in einem Temperaturbereich von 250 bis 400°C durchgeführt werden. Bei Verwendung eines γ-Aluminiumoxids als Katalysator beträgt die Temperatur im Reaktor vorzugsweise 300 bis 400°C, besonders bevorzugt. 300 bis 340°C, beispielsweise 330°C.

Anschließend wird eine beispielhafte Ausführungform des erfindungsgemäßen Verfahrens unter Einsatz von Methanol als Alkylierungsmittel beschrieben. Das Verfahren wird dreistufig durchgeführt.

Dazu wird Phenol über eine Dosierungseinrichtung in einen Mischer/Verdampfer geführt. Demselben Mischer/Verdampfer wird Methanol über eine Dosierungseinrichtung zugeführt. Das Gemisch der Ausgangsverbindungen wird in einen auf 330°C beheizten Rohrreaktor gespeist. Der Abstrom des Reaktors enthält neben den Produkten die Ausgangsverbindung Phenol und kann am Boden des Reaktors abgenommen werden. Der Abstrom wird in einen weiteren Mischer/Verdampfer eingeleitet, der einem weiteren Rohrreaktor vorgelagert ist. In diesem Mischer wird erneut das für das erfindungsgemäße Verfahren erforderliche Verhältnis Methanol/Phenol eingestellt und das erhaltene Gemisch in den zweiten Reaktor geleitet. Der Abstrom aus dem zweiten Reaktor wird entweder in einen Kühler oder in einen dritten Mischer/Verdampfer geleitet, der einem dritten Reaktor vorgeschaltet ist. In dem dritten Reaktor wird erneut das für das erfindungsgemäße Verfahren erforderliche Verhältnis Methanol/Phenol eingestellt und das erhaltene Gemisch in den dritten Reaktor eingespeist.

Der Abstrom aus dem dritten Reaktor, gegebenenfalls dem zweiten Reaktor, wird in einem Kühler kondensiert und das Kondensat einem Tank zugeführt.

Die Aufarbeitung des Rohalkylats kann vorzugsweise durch kontinuierliche Rektifikation in einem System aus drei hintereinander geschalteten Destillationskolonnen erfolgen.

In der ersten Kolonne mit beispielsweise 20 bis 35 praktische Böden, die bei Normaldruck betrieben wird, erfolgt die Abtrennung des Reaktionswassers am Kopf der Kolonne bei einer Kopftemperaturvon 90 bis 100°C. Das Wasser enthält in geringem Umfang Phenol, Alkylphenole und Anisol, da diese Verbindungen azeotrop mit dem Wasser destillieren.

Der Sumpf der ersten Kolonne wird kontinuierlich in die zweite Kolonne eingespeist. Diese Kolonne hat etwa 100 praktische Böden und wird ebenfalls bei Normaldruck betrieben. Am Kopf der Kolonne wird Phenol mit geringen Anteilen an o-Kresol bei einer Kopftemperatur 180 bis 185°C abgezogen. Dieser Mengenstrom kann der Alkylierungsstufe wieder als Rohstoff zugeführt werden.

Der phenolfreie Sumpf der Kolonne 2 gelangt kontinuierlich in die Einspeisung der dritten Kolonne mit beispielsweise mit 70 bis 95 praktischen Böden bei 300 mbar Kopfdruck und etwa 145 bis 155°C Kopftemperatur. Am Kopf der Kolonne kann reines o-Kresol einer Reinheit von > 99,5% abgezogen werden.

Der Sumpf der Kolonne, welcher geringe Anteile an o-Kresol enthält, kann als Rohstoff zur Herstellung von Kresol/Xylenolgemischen eingesetzt werden.

Erfindungsgemäß zu erhaltende o-alkylierte Verbindungen sind Kresol und die Ethyl-, n-Propyl-, Isopropyl-, n-Butyl- und Isobutylderivate des Phenols.

Die folgenden Beispiele dienen der weiteren Erläuterung der Erfindung.

### Versuch 1

Aus der Literatur ist bekannt, daß Anisol durch eine intramolekulare Umlagerung o-Kresol bildet. Aus diesem Grunde war davon auszugehen, im Verfahren eine hohe Konzentration von Anisol aufzubauen, um die Produktausbeute zu erhöhen. Dazu wurde in einem einstufigen Verfahren reines Anisol in einen Röhrenreaktor mit einer Temperatur von 330°C bei einer LHSV von 1,25 h⁻¹ eingeleitet. Als Katalysator wird ein γ-Aluminiumoxid mit einer Oberfläche von etwa 250 m²/g eingesetzt. Die erhaltenen Produkte und deren Konzentration im Produktgemisch sind in der folgenden Tabelle 1 dargestellt.

**TABELLE 1**

| **Verbindung** | **Konzentration %** |
|---|---|
| Anisol | 7,1 |
| Phenol | 32,2 |
| o-Kresol | 28,6 |
| 2,6-Xylenol | 16,1 |
| 2,3,6-Trimethylphenol | 4,0 |
| Pentamethylphenol | 3,3 |
| Anisolumsatz | 92,9% |
| o-Kresol-Selektivität | 30,8% |

Die Ergebnisse in der Tabelle zeigen, daß Anisol bei einem Umsatz von 92,9% unter den gewählten Bedingungen hochreaktiv ist. Die hohe Konzentration an Phenol und höher alkylierten Phenolen wie 2,6-Xylenol und 2,3,6-Trimethylphenol zeigt, daß nur ein Teil des Anisols in o-Kresol umgelagert wird. Der größte Teil des Anisols reagiert als Alkylierungsmittel.

Zur Bestätigung dieser Vermutung wurde ein Alkylierungsversuch unter denselben Bedingungen wie zuvor beschrieben unternommen, bei dem Methanol vollständig durch Anisol ersetzt wurde. Die erhaltenen Ergebnisse sind in der Tabelle 2 wiedergegeben.

**TABELLE 2**

| | **Alkylierungsmittel** | |
|---|---|---|
| | **Anisol** | **Methanol** |
| Verbindung | **Konzentration %** | **Konzentration %** |
| Anisol | -- | -- |
| Phenol | 76,8 | 72,3 |
| o-Kresol | 18,8 | 20,5 |
| 2,6-Xylenol | 2,6 | 3,8 |
| 2,3,6-Trimethylphenol | -- | 0,4 |
| o-Kresol-Selektivität | 81,0% | 74,0% |

Die Werte zeigen ein ähnliches Verhalten des Methanols wie des Anisols. Die Verwendung von Anisol zeigt sogar noch eine etwas höhere Selektivität als beim Einsatz von Methanol als. Alkylierungsmittel.

Es kann vermutet werden, daß die Selektivität für o-Kresol bei beiden Alkylierungsmitteln etwa gleich ist. Die etwas höhere o-Kresolselektivität des Anisols wird darauf zurückzuführen sein, daß parallel zur der Alkylierung des Phenols durch Anisol die Umlagerung von Anisol zu o-Kresol erfolgt.

Zur Untersuchung des Anteils an o-Kresol, der durch intramolekulare Umlagerung entstanden ist, wurde der gleiche Versuch mit 4-Methylanisol als Modellsubstanz durchgeführt. Die Konzentration der einzelnen Produkte im Produktgemisch [%] und der 4-Methylanisolumsatz sind in der Tabelle 3 angegeben.

**TABELLE 3**

| **Verbindung** | **Konzentration %** |
|---|---|
| Phenol | 57,7 |
| o-Kresol | 12,8 |
| p-Kresol | 18,6 |
| 2,6-Xylenol | 1,5 |
| 2,4-Xylenol | 6,4 |
| 4-Methylanisol-Umsatz | 100% |

Die Ergebnisse zeigen, daß neben o-Kresol signifikante Konzentrationen an p-Kresol und 2,4-Xylenol erhalten wurden. p-Kresol wird gebildet, wenn 4-Methylanisol als Alkylierungsmittel wirkt. 2,4-Xylenol ist das Produkt der intramolekularen Umlagerung von 4-Methylanisol. Die Berechnung zeigt, daß etwa 70% des 4-Methylanisols als Alkylierungsmittel und etwa 30% zu 2,4-Xylenol umgelagert werden. Es wird angenommen, daß beim Einsatz von Anisol als Alkylierungsmittel die gleichen Verhältnisse bestehen.

### Beispiel 1

Phenol wurde mit Methanol bei einer Reaktortemperatur von 330°C bei einem Molverhältnis Methanol/Phenol von 0,2 und einer LHSV von 3,75 h⁻¹ in den Reaktor gepumpt. Als Katalysator wird ein γ-Aluminiumoxid mit einer Oberfläche von etwa 250 m²/g eingesetzt. In der folgenden zweiten Stufe wurde Methanol im Molverhältnis 0,2 dem Abstrom des ersten Reaktors zugeführt und die Alkylierung fortgesetzt. In gleicher Weise wurde zur Durchführung der dritten Alkylierungsstufe verfahren.

Zum Vergleich wurde Phenol mit Methanol bei einer Reaktortemperatur von 330°C bei einem Molverhälnis Methanol/Phenol von 0,6 bei einer LHSV von 1,25 h⁻¹ in einem einstufigen Röhrenreaktor umgesetzt. Es wurde derselbe Katalysator eingesetzt. Insgesamt ist damit das Methanol/Phenol-Verhältnis und die LHSV dieselbe wie in der dreistufigen Reaktion. Die Produktkonzentration im Produktgemisch, der Phenolumsatz und die Selektivität für o-Kresol zu beiden Reaktionen sind in der Tabelle 4 dargestellt.

**TABELLE 4**

| **Verbindung** | **Einstufige Alkylierung** | **Dreistufige Alkylierung** |
|---|---|---|
| Anisol | 0,02 | 0,9 |
| Phenol | 53,7 | 58,6 |
| o-Kresol | 27,9 | 29,4 |
| m/p-Kresol | 1,5 | 0,8 |
| 2,6-Xylenol | 9,5 | 6,8 |
| 2,4/2,5-Xylenol | 2,0 | 0,9 |
| 2,3,6- Trimethylphenol | 1,7 | 0,9 |
| Phenolumsatz | 46,3 | 41,4 |
| o-Kresol-Selektivität | 60,3 | 71,1 |

Diese Ergebnisse zeigen, daß in dem mehrstufigen Verfahren die Selektivität für o-Kresol deutlich höher ist als in dem einstufigen Verfahren, obwohl der Phenolumsatz in dem dreistufigen Verfahren ähnlich ist. Der Grund liegt darin, daß die Menge der Nebenprodukte im einstufigen Verfahren deutlich höher ist als im dreistufigen Verfahren. Obwohl der Anisolgehalt von 0,9% in diesem Versuch noch nicht optimal ist, wird eine Steigerung der Selektivität für o-Kresol von 60,3% beim einstufigen Verfahren auf 71,1% im dreistufigen Verfahren erhalten.

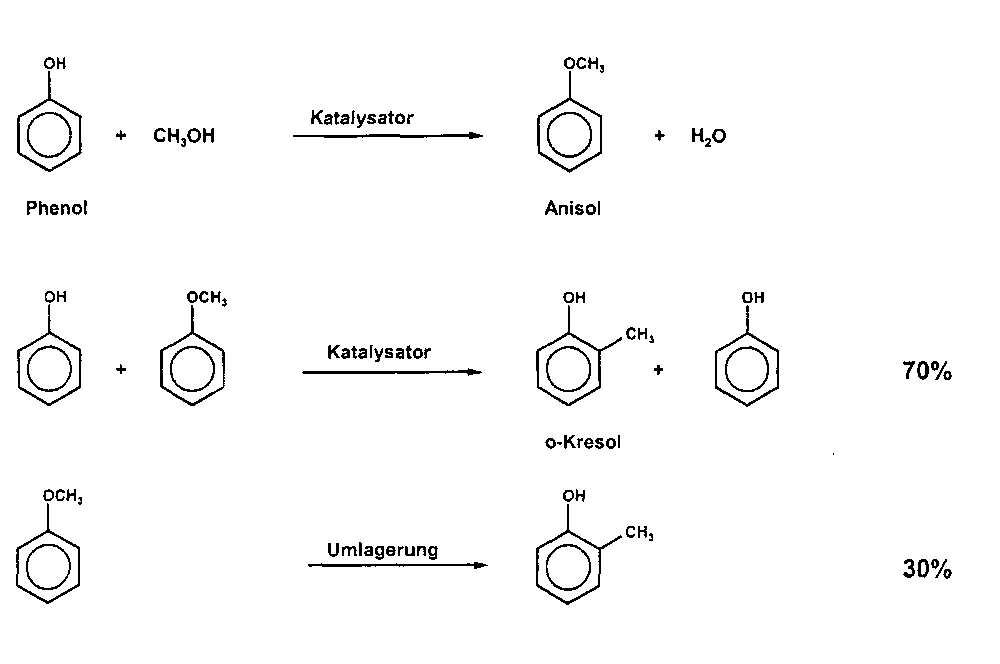

## Patentansprüche

1. Verfahren zur Herstellung von o-Alkylphenolen durch Umsetzen von Phenol mit einem Alkanol bei erhöhter Temperatur in der Gasphase in Gegenwart eines Metalloxidkatalysators, **dadurch gekennzeichnet, dass** man die Umsetzung in mindestens zwei Stufen durchführt und das Molverhältnis Alkanol/Phenol in jeder Reaktionsstufe auf einen Wert von etwa ≤ 0,4 einstellt.

2. Verfahren nach Anspruch 1, **dadurch gekennzeichnet, daß** man die Umsetzung in drei Stufen durchführt.

3. Verfahren nach Anspruch 1 oder 2, **dadurch gekennzeichnet, daß** man das Molverhältnis Alkanol/Phenol in jeder Reaktionsstufe auf einen Wert von etwa 0,2 bis 0,4 einstellt.

4. Verfahren nach Anspruch 3, **dadurch gekennzeichnet, daß** man das Molverhältnis Alkanol/Phenol in jeder Reaktionsstufe auf einen Wert von etwa 0,3 einstellt.

5. Verfahren nach einem der Ansprüche 1 bis 4, **dadurch gekennzeichnet, daß** man den Phenolumsatz während der Reaktion in jeder Stufe auf 35 bis 43% einstellt.

6. Verfahren nach einem der Ansprüche 1 bis 5, **dadurch gekennzeichnet, daß** man als Alkanol Methanol einsetzt.

7. Verfahren nach einem der Ansprüche 1 bis 6, **dadurch gekennzeichnet, daß** man als Katalysator ein γ-Aluminiumoxid mit einer Oberfläche von größer als 250 m²/g einsetzt.

8. Verfahren nach Anspruch 7, **dadurch gekennzeichnet, daß** man die Reaktion in einem Temperaturbereich von 300 bis 400°C durchführt.

9. Verfahren nach einem der Ansprüche 1 bis 8, **dadurch gekennzeichnet, daß** man das nach der Alkylierung erhaltene Produktgemisch durch Destillation zur Gewinnung des gewünschten Produkts trennt.

## Claims

1. A method for the production of o-alkyl phenols by conversion of phenol with an alkanol at elevated temperature in the gas phase in the presence of a metal catalyst, **characterised in that** the conversion is carried out in at least two stages and the alkanol/phenol molar ratio in each reaction stage is set to a value of approximately ≤ 0.4.

2. The method according to Claim 1, **characterised in that** the conversion is carried out in three stages.

3. The method according to Claim 1 or 2, **characterised in that** the alkanol/phenol molar ratio in each reaction stage is set to a value of approximately 0.2 to 0.4.

4. The method according to Claim 3, **characterised in that** the alkanol/phenol molar ratio in each reaction stage is set to a value of approximately 0.3.

5. The method according to one of Claims 1 to 4, **characterised in that** the phenol conversion during the reaction is set to 35 to 43 % in each stage.

6. The method according to one of Claims 1 to 5, **characterised in that** methanol is used as alkanol.

7. The method according to one of Claims 1 to 6, **characterised in that** an γ-aluminium oxide having a surface area greater than 250 m²/g is used as the catalyst.

8. The method according to Claim 7, **characterised in that** the reaction is carried out in a temperature range of 300 to 400 °C.

9. The method according to one of Claims 1 to 8, **characterised in that** the product mixture obtained after alkylation is separated by distillation to obtain the desired product.

## Revendications

1. Procédé de préparation de O-alkylphénols par réaction du phénol avec un alcanol, à température élevée en phase gazeuse, en présence d'un catalyseur d'oxyde métallique, **caractérisé en ce que** l'on réalise la réaction en au moins deux étapes, et que le rapport molaire alcanol/phénol est ajusté dans chaque étape de réaction, à une valeur environ ≤ 0,4.

2. Procédé suivant la revendication 1, **caractérisé en ce que** l'on réalise la réaction en trois étapes.

3. Procédé suivant la revendication 1 ou 2, **caractérisé en ce que** l'on ajuste le rapport molaire alcanol/phénol dans chaque étape de réaction, à une valeur allant d'environ 0,2 à 0,4.

4. Procédé suivant la revendication 3, **caractérisé en ce que** l'on ajuste le rapport molaire alcanol/phénol dans chaque étape de réaction, à une valeur d'environ 0,3.

5. Procédé selon l'une quelconque des revendications 1 à 4, **caractérisé en ce que** l'on ajuste la transformation du phénol pendant la réaction, à chaque étape, dans l'intervalle allant de 35 à 43%.

6. Procédé selon l'une quelconque des revendications 1 à 5, **caractérisé en ce que** l'on met en oeuvre le méthanol comme alcanol.

7. Procédé selon l'une quelconque des revendications 1 à 6, **caractérisé en ce que** l'on met en oeuvre comme catalyseur, un γ-oxyde d'aluminium avec une surface supérieure à 250 m²/g.

8. Procédé suivant la revendication 7, **caractérisé en ce que** l'on réalise la réaction dans un intervalle de température allant de 300 à 400°C.

9. Procédé selon l'une quelconque des revendications 1 à 8, **caractérisé en ce que** l'on sépare le mélange de produits obtenu après l'alkylation, par distillation pour obtenir le produit souhaité.
